# EUROPEAN PATENT SPECIFICATION

(11) **EP 0 434 721 B1**
(45) Date of publication and mention of the grant of the patent: **15.03.2000**
(21) Application number: 89910146.3
(22) Date of filing: 13.09.1989
(51) Int. Cl.: C12N 15/38, C12N 15/86, A61K 39/245, A61K 39/255

(54) **VIRAL NUCLEOTIDE SEQUENCES**
VIRALE NUCLEOTIDSEQUENZEN
SEQUENCES DE NUCLEOTIDES VIRALES

(30) Priority: 13.09.1988 GB 8821441
(43) Date of publication of application: 03.07.1991
(60) Divisional application: 99114400.7
(73) Proprietor: MERIAL, 69002 Lyon (FR)
(72) Inventor: GRIFFIN, Annette, Mary Inst.for Animal Health Ltd., Huntingdon PE17 2DA (GB); ROSS, Louis, J.N. Institute for Animal Health Ltd., Huntingdon PE17 2DA (GB); SCOTT, Simon, David Inst.for Animal Health Ltd., Huntingdon PE172DA 7 (GB); BINNS, Matthew, M. Inst.for Animal Health Ltd., HuntingdonPE17 2DA (GB)
(74) Representative: Bernasconi, Jean
(86) International application number: PCT/GB89/01075
(87) International publication number: WO 90/02802

(56) References cited:
- WO-A-87/04463
- J. Gen. Virol., Volume 69, 1988, SGM, (GB), A.E. BUCKMASTER et al.: "Gene Sequence and Mapping Data from Marek's Disease Virus and Herpesvirus of Turkeys: Implications for Herpesvirus Classification", pages 2033-2042
- CHEMICAL ABSTRACTS, Volume 110, No. 17, 24 April 1989, (Columbus, Ohio, US), see page 231* Abstract 149170c, & US, A, 128836 (United States Dept. of Agriculture) 15 July 1988*

## Description

The present invention relates to the DNA fragment encoding the HVT 2B gene and its use in the preparation of vaccines.

### Background and Description of prior art

Herpesviruses are large double stranded DNA viruses consisting of an icosahedral capsid surrounded by an envelope. The group has bean classified as alpha, beta and gammaherpesviruses on the basis of genome structure and biological properties [Roizman, B et al (1981) Intervirology 16, 201-217]. Avian herpes viruses include Marek's Disease Virus (MDV) (a gammaherpesvirus) which causes a lymphomatous disease of considerable economic importance in chickens [reviewed in Payne, L.N. (ed) Marek's Disease (1985), Martinus Nijhoff Publishing, Boston) and Infectious Laryngotracheitis Virus (ILTV) (an alphaherpesvirus) which causes an acute upper respiratory tract infection in chickens resulting in mortality and loss of egg production.

A recent unexpected finding in out laboratory is that there is sufficient amino acid homology between MDV, ILTV and mammalian herpesviruses, particularly varicella zoster (VZV) and Herpes Simplex Virus (HSV) to allow identification of numerous conserved genes. These include the MDV and Herpesvirus of Turkeys (HVT) homologues of glycoproteins gB, gC and gH of HSV; the ILTV, MDV and HVT homologues of TK and ribonucleotide reductase genes and the ILTV homologue of gB and genes 34 and 35 of VZV [Buckmaster, A et al, (1988) J. gen. Virol, 69, 2033-2042.

Strains of MDV have been classified into three serotypes. Type 1 comprises pathogenic strains and their attenuated derivatives. Type 2 are a group of naturally-occurring non-pathogenic strains and type 3 is HVT. For more than a decade, vaccination with HVT has been remarkably effective in controlling Marek's disease. However, in recent years, new strains of MDV have been isolated which cause disease despite vaccination with HVT. Losses due to these 'very virulent' strains have occurred in parts of the U.S.A., Europe and the Middle East. Although the degree of protection can be improved by using a mixture of HVT, type 2 MDV and attenuated derivatives of very virulent strains for vaccination, the results have been erratic. These observations and the fact that there are MDV type-specific epitopes that are not shared by HVT or type 2 MDV have led us to the conclusion that improved vaccines might be constructed which are antigenically more related to MDV than existing vaccines. [Reviewed by Ross and Biggs in Goldman J.M. and Epstein M.A. (eds) Leukaemia and Lymphoma Research, Vaccine Intervention against Virus-Induced Tumour, p 13-31, Macmillan, 1986].

Infectious laryngotracheitis is also a worldwide problem. Sporadic outbreaks occur in which the severity of clinical symptoms varies considerably. Virus can persist in birds that have recovered and may be shed at intermittent intervals after recovery. An attenuated field strain is currently used as a vaccine. However, it has retained some degree of pathogenicity. Mortality due to the vaccine may reach 10% in young chicks.

A number of herpesvirus antigens have been shown to confer protective immunity when expressed in a recombinant vaccinia virus. These include the gB gene of HSV [Cantin E.M. et al (1987) Proc. Natl. Acad. Sci. U.S.A. 84, 5908-5912], gD of HSV [Paoletti, E. et al (1984) Proc. Natl. Acad. Sci. U.S.A. 81, 193-197] and gp50 of pseudorabies virus (PRV), a homologue of HSV gD [Marchioli, C.C. et al (1987) J. Virol. 61, 3977-3981]. Because of the absolute requirement of gB for virus penetration and infectivity and because it is conserved among herpes-viruses, gB and its homologues are important immunogens. Moreover, the presence of gB at the surface of infected cells has been shown to be an important target for humoral and cell-mediated immune responses [Blacklaws, BA. et al J.gen. Virol. 68. 1103-1114 (1987); McLaughlin-Taylor, E. et al (1988) J. gen. Virol. 69, 1731-1734). The recently described glycoprotein gH of HSV is also essential for infectivity and may also be an important immunogen [Desai, P.J. et al (1988) J. gen. Virol. 69, 1147-1156]. It has also been shown that gIII of pseudorabies virus (PRV), a homologue of gC, is a major target for neutralizing antibody and for cytotoxic T cells although it is a non-essential protein. Also of interest is the unexpected participation of immediate early proteins in T cell mediated cytotoxic reactions in cells infected with cytomegalovirus (CMV) [Kozinowski U.H. et al (1987) J. Virol. 61, 2054-2058]. Similar antigens could play an important role in the rejection of latently infected and transformed lymphocytes in Marek's disease since immediate early RNA transcripts have been detected in lymphoblastoid cell lines established from Marek's disease tumours.

Although many recombinant vaccines have been constructed using the poxvirus vaccinia as a vector, there are also reports of the use of herpesviruses as vectors for the expression of foreign genes. Thus hepatitis antigen has been expressed in HSV [Shih, M.F. et al (1984) Proc. Natl. Acad. Sci. U.S.A. 81, 5867-5870] and human tissue plasminogen activator has been expressed in PRV [Thomsen, D.R. et al (1987) Gene 57, 261-265. In both cases, foreign genes were inserted in cloned fragments of non-essential herpes genes which were then introduced into the virus vector by homologous recombination. The hepatitis virus gene was fused to a herpesvirus promoter and the recombinant DNA was inserted within the TK gene of HSV. Homologous recombination following co-transfection of the recombinant DNA and wild-type HSV DNA resulted in TK⁻ virus clones that expressed the hepatitis antigen.

In the case of PRV, the gX gene mapping in Uₛ was used as the site for insertion of the foreign gene. The strategy used involved insertion of the TK gene of HSV in the gX gene of a PRV mutant that had a defect in its TK gene resulting in a TK positive virus. The human tissue plasminogen activator gene was then inserted within a cloned fragement of HSV TK and the recombinant was introduced into the PRV mutant by homologous recombination. TK⁻ virus was selected which expressed the human gene (Thomsen et al as above). Similarly, VZV has been used as a vector [Lowe et al (1987) Proc. Natl. Acad. Sci. U.S.A. 84, 3896-3900].

Several herpesvirus genes have also been shown to be associated with virulence and to be non-essential for growth in vitro. These include the TK genes of HSV [Jamieson, A.T. et al (1974) J. gen. Virol. 24, 465-480; Field, H. and Wildy, P., (1987) J. Hygiene (Cambridge) 81, 267-277] and of PRV. Indeed it has long been known that PRV is readily attenuated by deletion of TK activity [Tatarov, G. (1968) Zentralbl. Vet. Med 15B, 848-853]. Furthermore, attenuation of the Bartha strain of PRV has been attributed to a defect in gI, a non-essential structural glycoprotein mapping in Uₛ [Mettenleiter, T. et al (1987) J. Virol. 61, 4030-4032].

Genes of HSV mapping in the internal repeat region (TRS) flanking the long unique sequence have also been associated with pathogenicity [Rosen, A. et al (1986) Virus Research 5, 157-175; Thompson, R.L. et al (1983) Virology 131, 180-192]. Several additional genes of HSV have been shown to be non-essential for growth in vitro although it is not known whether they are associated with virulence. These include UL24 (Sanders, P.G., (1982), J. gen. Virol. 63, 277-295, large subunit of ribonucleotide reductase (Goldstein D.J. and Weller, S.K. (1988) J. Virol. 62, 196-205), gC (Draper K.G. et al (1984) J. Virol. 51, 578-585), dUTPase (Fisher, F.B. & Preston, V.G. (1986) Virology 148, 190-197), and U_{L}55 and U_{L} 56 (MacLean, A.R. & Brown, S.M. (1987) J. gen. Virol. 68, 1339-1350).

Moreover there is evidence that several genes of HSV mapping in Uₛ are also non-essential for growth in vitro [Weber, P.C. et al (1987) Science 236, 575-579].

### Summary of the invention

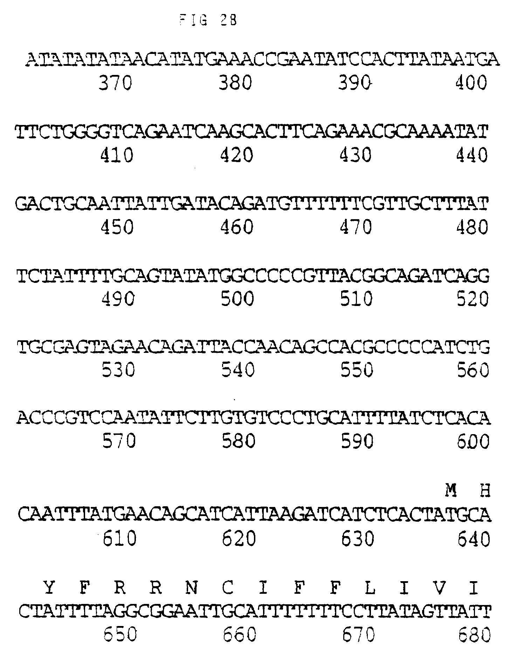

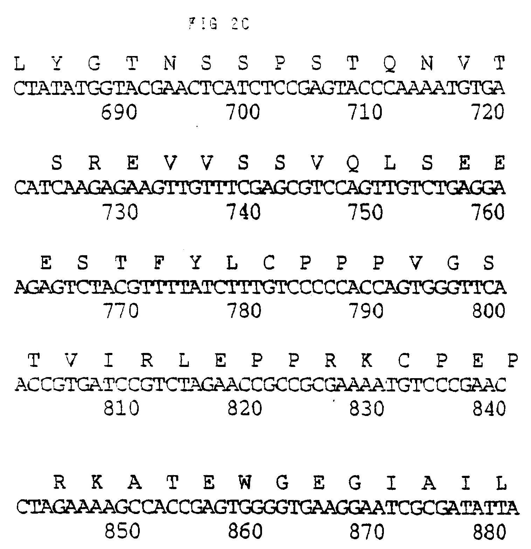

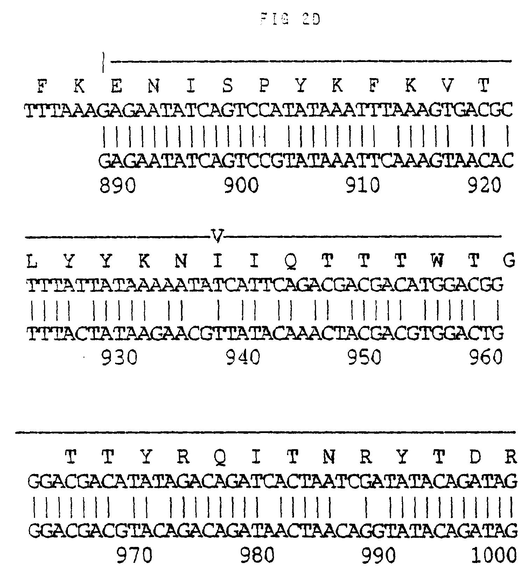

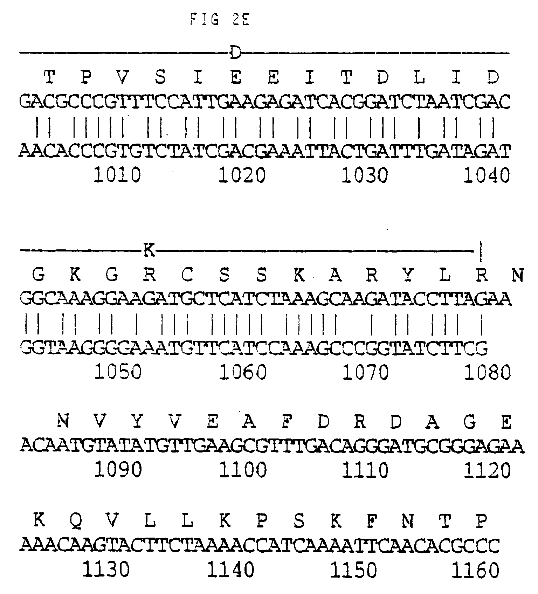

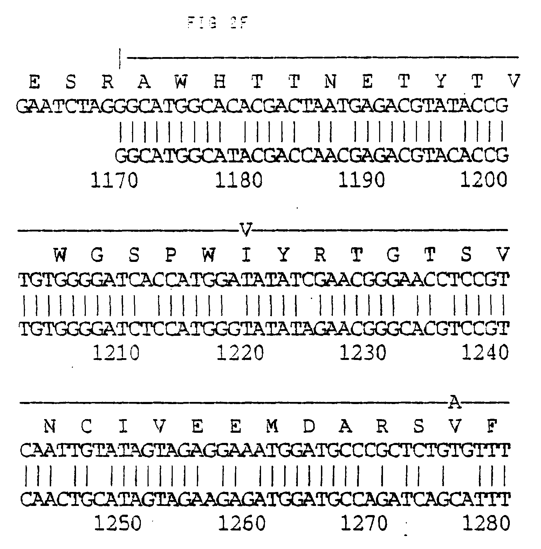

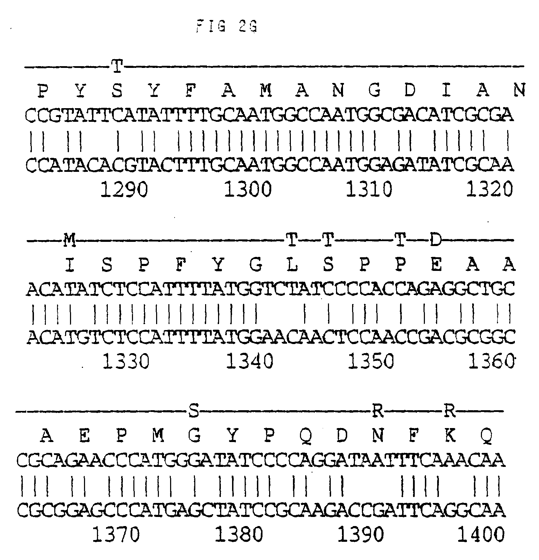

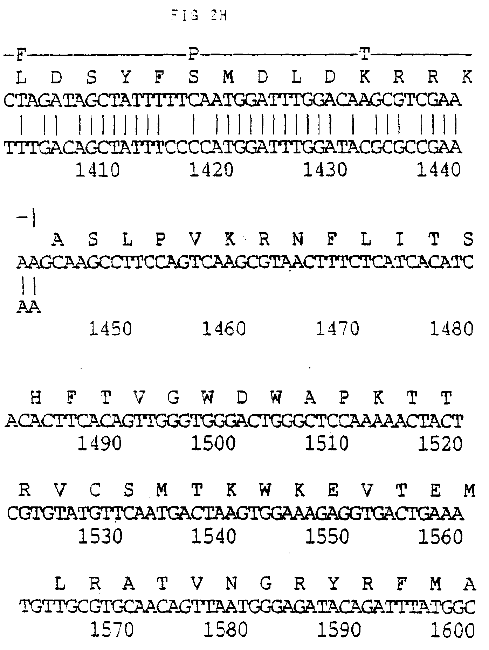

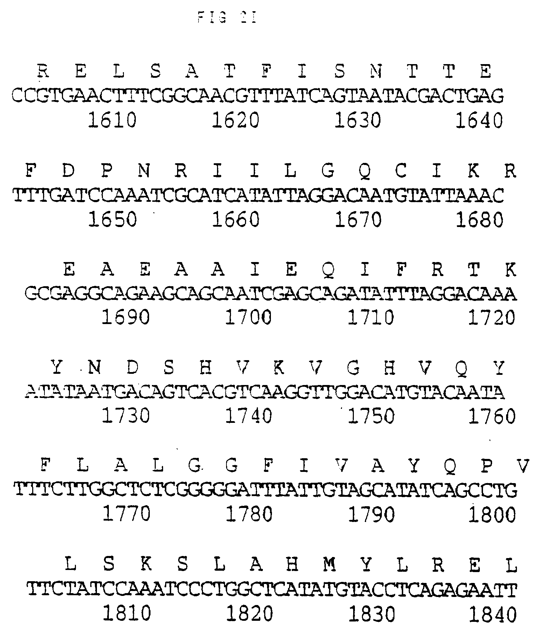

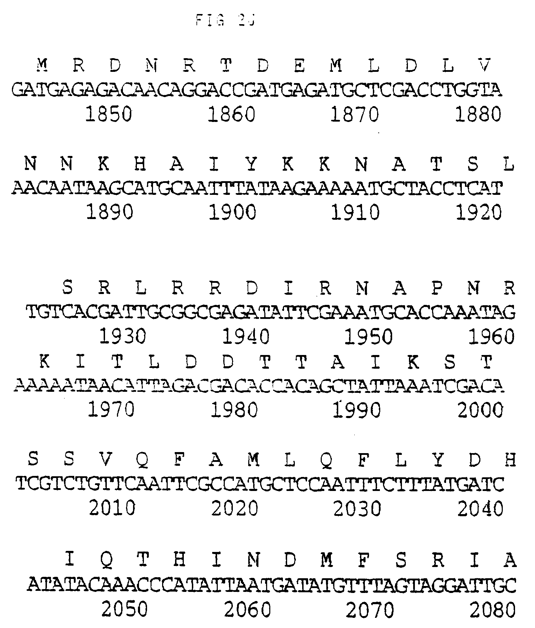

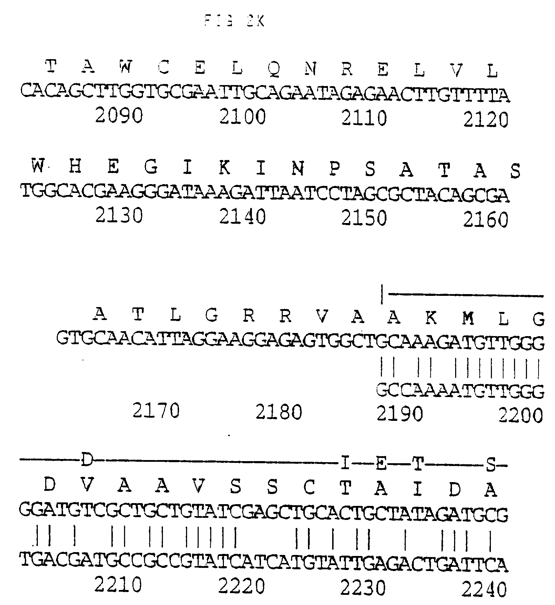

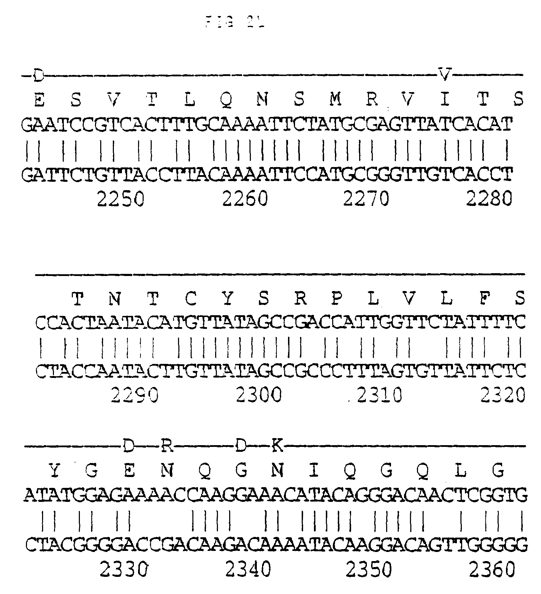

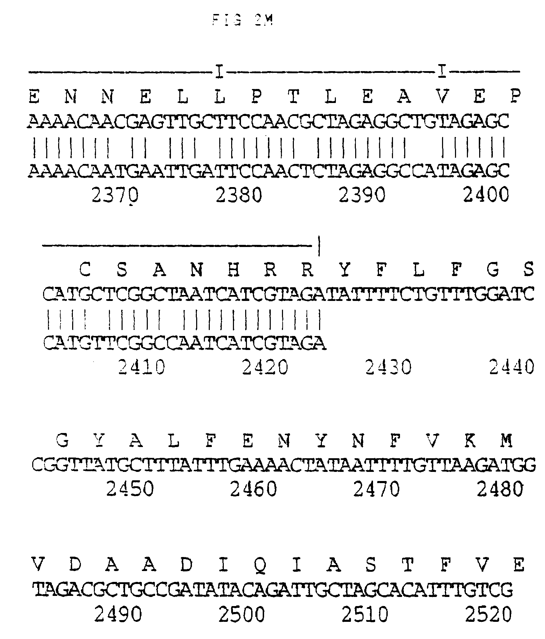

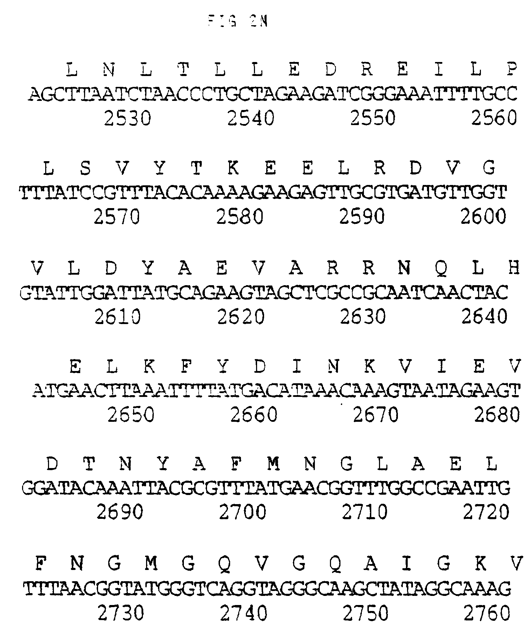

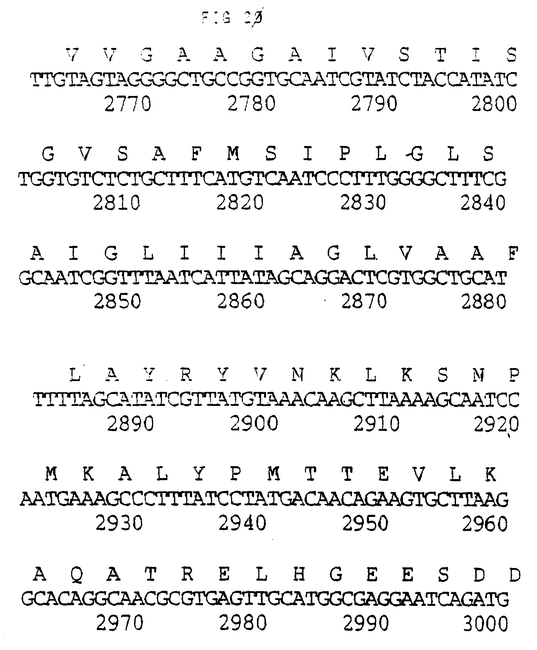

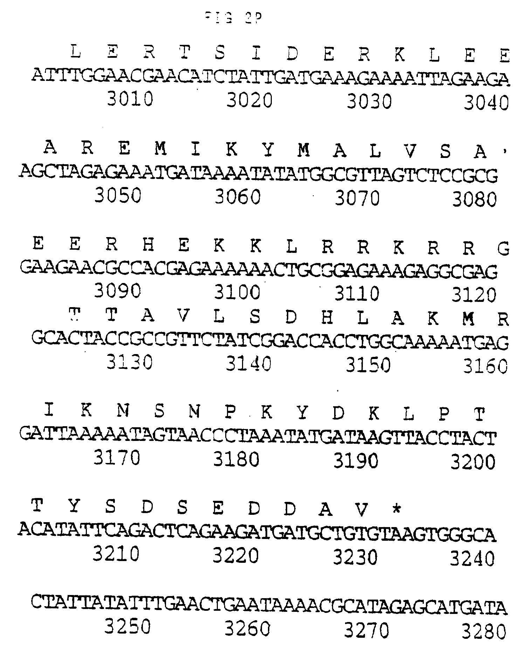

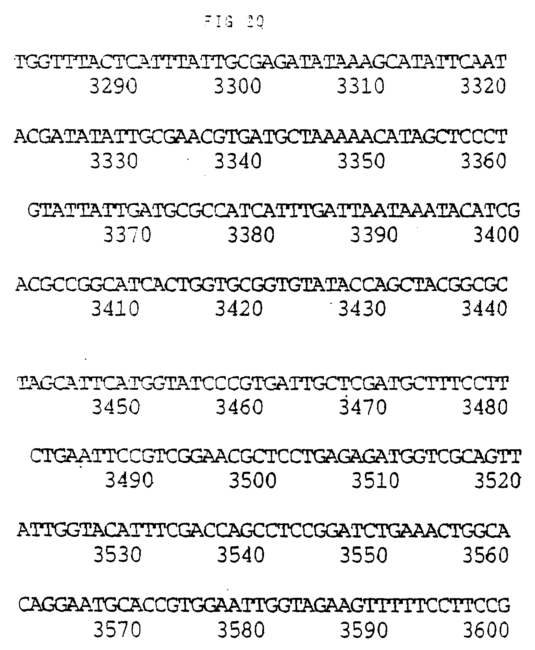

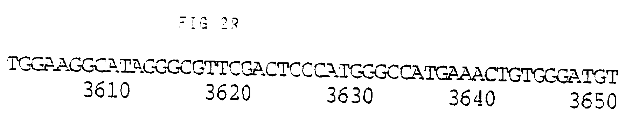

One object of the present invention is a DNA fragment encoding the HVT gB gene which comprises the HVT nucleotide sequence portions of Figure 2 indicated between nucleotide positions 890-1081, 1172-1445 and 2190-2426 or minor variations thereof.

The sequence may be associated with further elements such as suitable stop and start signals and other 5' and 3' non-coding sequences, including promoters, enabling expression of the sequence. Such further elements may be those associated with the sequence in its naturally-occurring state or may be heterologous to that sequence.

So, the DNA fragment according to the invention may comprise the coding portion of the gB gene sequence and at least part of the 5' and/or 3' non-coding portions thereof.

The term "minor variations thereof" is intended to include changes in the nucleotide sequence which do not effect its essential nature, for example minor substitutions of nucleo-tides for one another. The "essential nature" or the sequence refers to the (glyco)protein encoded. Conservative changes in the nucleotide sequence which give rise to the same antigen will clearly be included, as will changes which cause conservative alterations in the amino acid sequence which do not affect adversely the antigenic nature of the antigen, in particular, antigenic portions of the antigen sequences may be used alone.

The DNA fragment according to the invention is useful for expressing viral antigens.

The DNA fragment may readily be isolated from naturally-occurring HVT viruse, using the sequence information given herein and standard techniques, for example involving the preparation of oligonucleotide probes and use thereof to hybridise to the naturally-occurring DNA.

The DNA fragment according to the invention may be expressed in any suitable host and, in particular, in HVT or MDV.

A second aspect of the invention provides insertional or deletional mutants of MDV, HVT and ILTV as follows:
a mutant MD or ILT virus comprising a DNA fragment encoding the HVT gB gene as described above, which is inserted in the gC, ribonucleotide reductase (small subunit) or ribonucleotide reductase (large subunit) gene of MDV or in the TK, ORF2, ORF3 or ribonucleotide reductase (large subunit) gene of ILTV, or a mutant HVT virus comprising an extra copy of the said DNA fragment, inserted in the gC, ribonucleotide reductase (large subunit) or TK gene of HVT.

Each mutation may be in the coding or non-coding sequences of the regions identified.

Such mutant forms of HVT, MDV and ILTV may be used as, or created in the course of preparing, viral vectors for the heterologous antigen-encoding DNA fragment.

By "heterologous", we mean that the antigen-expressing sequence has not previously been found in the same place in relation to the remainder of the viral genome.

A convenient process for preparing the deletional or insertional mutants of the second aspect of the invention comprises simply introducing into a suitable cell, for example by co-transfection, a deletional or insertional mutant version of the appropriate region (for example. the TK region) and either whole viral DNA or a whole virus (for example the wild-type virus). The naked DNA of such viruses has been found to be infectious, provided that it has not been sheared. A calcium phosphate precipitate of the DNA is generally advantageous. Suitable cells include chicken embryo fibroblasts, chicken kidney cells and duck embryo fibroblasts, all preferably grown in sub-confluent monolayers in Petri dishes.

The transfected DNA and the whole viral DNA will then recombine with one another in the infected cells br homologous recombination and the desired recombinants can be screened for, for example by the detection of hybridisation to suitable probes or by art immunoassay using suitable antibodies to the gene product of the region in question.

For homologous recombination to take place, the viral DNA must replicate. At present, no cell-free replication system for MDV, HVT or ILTV is known. However, if such a system becomes available, then the process of the invention could be operated therein. The environment in which the replication and recombination occur is not critical.

The HVT region which was identified above as being responsible for encoding immunologically useful viral antigens can be inserted into suitable vectors, for example into HVT or into other vectors such as fowlpoxvirus, bacteria or fungi. In the case of viral vectors, especially herpesvirus vectors and poxvirus vectors, such insertion can be achieved by recombination betwen the antigen-encoding sequence, flanked by suitable non-essential sequences, and the vector's genome in a suitable host cell as described above. A promoter which is endogenous to the host will usually be used to control expression of the heterologous (viral antigen-encoding) sequence. In the case of bacteria and fungi, the antigen-encoding sequence may be inserted using known or yet-to-be-discovered techniques of DNA manipulation. A non-pathogenic strain of Salmonella may he used as such a host. The heterologous sequence may be inserted into the host's genome or be carried on an independently-replicating plasmid.

The flanking sequences which are used may comprise all, virtually all or less of the region into which the heterologous sequence is to be inserted. If all the region is employed, then the sequence of that region will clearly still be present in the resulting virus, but the function of that region will have been deleted. If less than the whole region is used as flanking sequences, then the result will be a structural as well as functional deletion. Either approach may be used.

To prepare a vaccine in which HVT, MDV or ILTV is the virus or vector, the virus is grown in suitable cells such as chick embryo fibroblasts in a standard culture medium which as 199 medium (Wellcome or Flow Laboratories) for 3 to 4 days at about 37°C. The cells are harvested by scraping from the surface of the culture or by trypsinisation and suspended in medium containing 1mM EDTA or 10% dimethyl sulphoxide and in either case 4% calf serum before storage in liquid nitrogen in sealed ampoules.

For vaccination, typically, day-old chicks are injected intramuscularly with about 1,000 plaque-forming units. Immunity follows within a few days.

It should be noted that MDV and HVT are cell-associated viruses and are infectious only when present in cells. Thus, a vaccine based on such viruses will always include suitable infected cells.

The vaccines of the invention may be used to protect any fowl susceptible to HTV, including commercially-reared poultry such as chickens, turkeys, ducks and quail.

Preferred aspects of the invention will now be described by way of example and with reference to the accompanying drawings, in which:
Figure 1 is a map of the MDV genome showing in part the BamH1 site distribution and the location of the gB and TK genes;
Figure 2 (on 18 sheets) shows the nucleotide sequence of the gB gene of the RBIB strain of MDV, with the numbering referring to the MDV nucleotides, the sequence of part of the HVT gB gene shown under the line, homologies indicated by vertical bars, and amino acid differences between MDV gB and HVT gB shown above the line;
Figure 3 is a map of the HVT genome showing the positions of the gH (hatched), TK (solid black) and major capsid protein (MCP, dotted) genes, with HindIII sites shown as "H";
Figure 4 (on 10 sheets) shows the nucleotide sequence of the HVT TK gene, with the numbering referring to the HVT nucleotides, the sequence of part of the MDV TK gene shown under the line, homologies indicated by vertical bars and amino acid differences between MDV TK and HVT TK shown above the line;
Figure 5 (on 6 sheets) shows the nucleotide sequence of the gC gene of the RBIB strain of MDV, with corresponding amino acids shown above the line;
Figure 6 (on 11 sheets) shows the nucleotide and predicted amino acid sequence of a 5400 base pair region of the ILTV genome containing the TK gene cluster. Amino acid sequences predicted for the products of the major open reading frames (ORFs) are indicated in the single letter code below the sequence for the strand and above the sequence for the complementary strand. The locations of potential 'TATA' boxes are underlined. ORF 4 is the ILT TK gene sequence;
Figure 7 is a representation of the gene organisation in the TK-containing part of the ILTV genome. Overlapping pUC 13 plasmid clones containing the EcoR1 (pILEc1) and BglII (pILBg2) generated fragments of ILTV DNA are indicated. Open reading frames (ORFs) are depicted as open boxes with the direction of transcription indicated by the arrow;

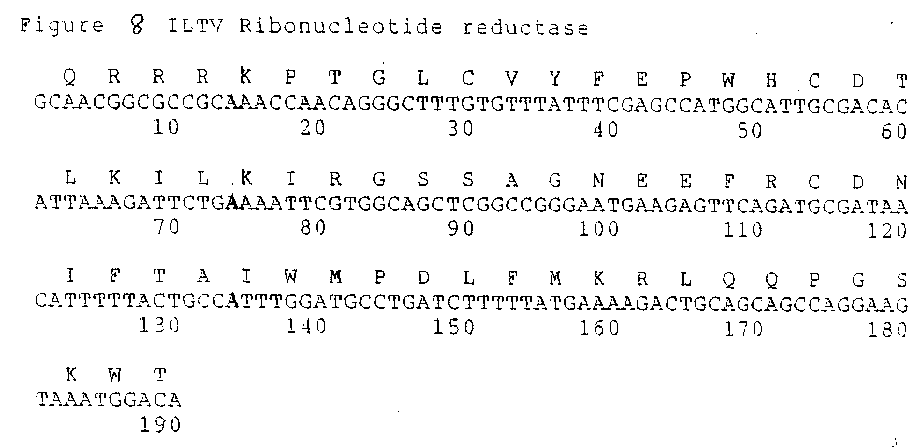

Figure 8 shows part of the nucleotide sequence of the ILTV ribonucleotide reductase (large subunit);

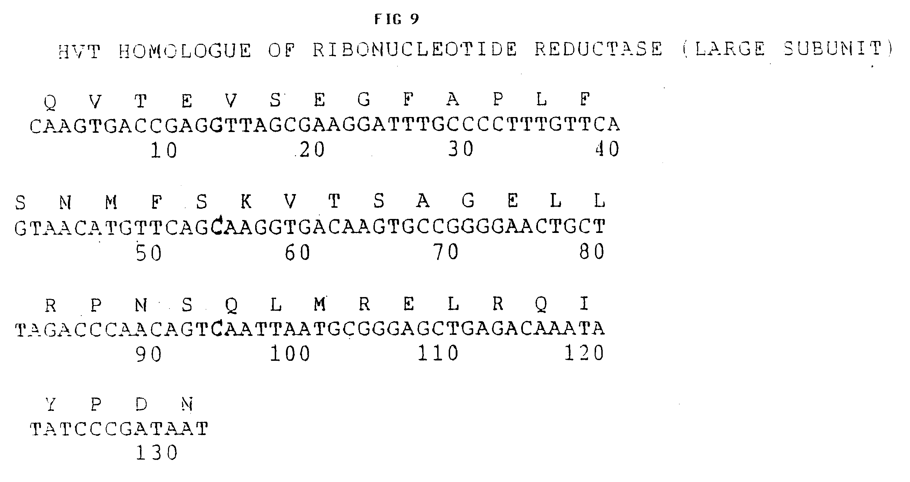

Figure 9 shows part of the nucleotide sequence of the HVT ribonucleotide reductase (large subunit) gene;

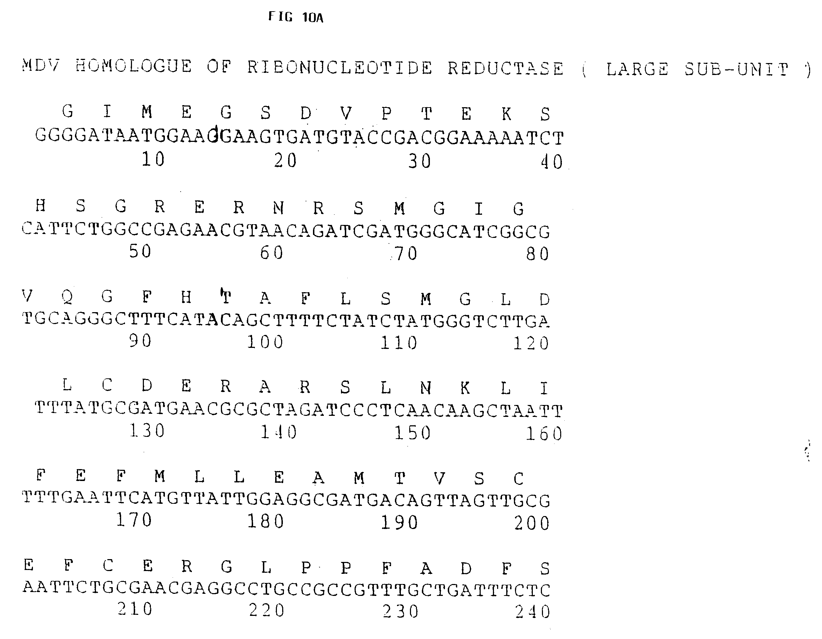

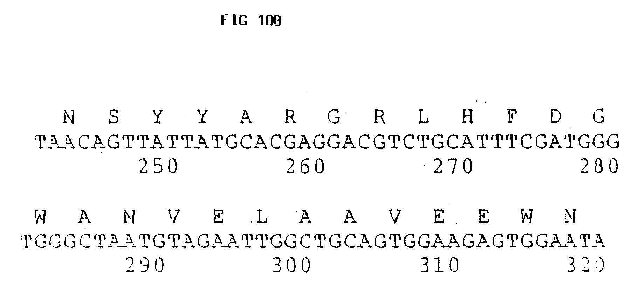

Figure 10 (on 2 sheets) shows part of the nucleotide sequence of the MDV ribonucleotide reductase (large subunit) gene;

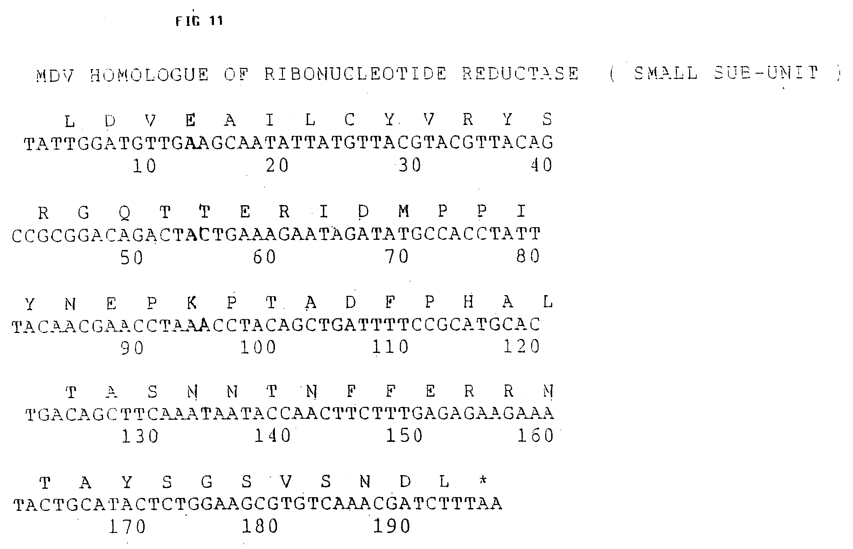

Figure 11 shows part of the nucleotide sequence of MDV homologue of ribonucleotide reductase (small subunit) gene;
Figure 12 is a map of plasmid pILBg2, showing restriction sites and the locations of the TK gene and ORFs 3 and 5.

### EXAMPLES: General Approaches

Selected short sequences of the avian herpesviruses cloned in the bacteriophage vector M13 were used as probes to identify longer fragments that might contain the entire genes of interest. This was achieved by Southern blot hybridization of restriction fragments. Full details are given below.

Virus Strains. The 'highly oncogenic' strain RB1B of MDV [Schat, K.A. et al (1982) Avian Pathol. 11, 593-605] was obtained from Professor B. Calnek, Cornell University, Ithaca, U.S.A. The virus received has been plaque purified in chicken kidney veils in tissue culture. It was passaged twice in SPF RIR chickens and 4 times in chick embryo fibroblasts (CEF). Its 'highly oncogenic' nature was demonstrated by a high incidence of gross tumours when inoculated in genetically resistant N-line chickens.

The FC126 strain of HVT [Witter, R.L. et al (1970) Am. J. Vet. Res. 31, 525-538), obtained from the Wellcome Research Laboratories, Beckenham, Kent, had been passaged 14 times in CEF. It was subsequently grown in duck embryo fibroblasts (DEF) and CEF in our laboratory. It was then plaque-purified and grown further in CEF. Viral DNA used for cloning in the present work was extracted from virus that had been passed 29 times since the original isolation.

The Thorne strain of ILTV was passaged twice in eggs, once in chicken kidney cells (CKC) and plaque-purified three times in CKC.

Tissue culture. CEF were grown in roller bottles in 199 medium (Wellcome), supplemented with penicillin, streptomycin, Fungizone (Regd. T.M.) and calf serum as described previously [Ross, L.J.N. et al (1975) J. gen. Virol. 28, 37-47).

CKC were grown in 10 cm Petri dishes [Churchill, A.E. and Biggs P.M., (1967) Nature, 215, 528-530].

Isolation of MDV DNA. Cell associated RB1B was inoculated onto confluent monolayers of CEF in roller bottles at a multiplicity of infection of approximately 0.001 plaque-forming units (pfu) per cell, and the cultures were incubated at 37°C. After 3 days, the medium was discarded and replaced with fresh 199 medium containing 2% calf serum. Cells were harvested for virus purification after 2 to 3 days when cytopathic effect was extensive. Virus was obtained by rate zonal centrifugation of the cytoplasmic fraction of infected cells [Lee, Y.S. et al (1980) J. gen. Virol. 51, 245-253]. Viral DNA was extracted by treating purified virus with sarcosyl, proteinase K and Tris buffer pH 9 overnight at 37°C and purified by rate zonal centrifugation in glycerol gradients as described previously (Lee et al, 1980). High molecular weight viral DNA was precipitated with ethanol and resuspended in 10 mM Tris pH 7.5 im 1mM EDTA (TE).

Isolation of ILTV DNA. (a) Infected CKC were harvested 2-3 days after inoculation, washed in PBS, and resuspended in ice-cold TE by vortexing. Cells were lysed by addition of the non-ionic detergent NP40 (final 1%) vortexing and incubation on ice for 15 min. After treatment with RNAse, the preparation was centrifuged at 2000 rpm for 5 min in a bench top centrifuge (Centaur). The supernatant was collected and incubated at 37°C for 30 min in the presence of SDS (final 1%) and proteinase K (final 0.5 mg/ml). The mixture was extracted twice with phenol-chloroform and once with chloroform and the DNA was then precipitated with ethanol and 1/10 vol of 3M sodium acetate.
(b) Viral DNA was also isolated from the media of virally infected cells in the following way. The media of infected cells were harvested at 2-3 days post infection and centrifuged at 3000 for 5 wins at 4°C rpm in a bench centrifuge. The supernatant was collected and centrifuged at 19K rpm in an ultracentrifuge (Sorvall) for 1 hr at 4°C. The viral pellet was resuspended in TE, digested with RNAse A, then disrupted with SDS and proteinase K as described above. Finally, DNA was extracted from the disrupted virus as described above.

Cloning of MDV DNA. One *f*g of MDV DNA was cut with the restriction enzyme BamH1 and ligated to BamH1-cut, dephosphorylated pUC13 DNA (Pharmacia). Competent E.coli strain TG1 cells were transformed according to standard procedures [Hanahan, D. (1983) J. Mol. Biol. 166, 557-580] and were grown in the presence of ampicillin and X-gal. White colonies were picked and tested for the presence or MDV inserts by hybridization to nick-translated MDV DNA [Grunstein M. and Hogness, D.S. (1975) Proc. Natl. Acad. Sci. U.S.A. 72, 3961]. Positive colonies were cultured in small volume and plasmid DNA isolated by the procedure of Holmes, D.S. and Quigley, M. [(1981) Anal. Biochem. 114, 193-297]. The size of the inserts was determined by electrophoresis of BamH1 digests of the recombinant DNA in agarose gels. Plasmids containing MDV inserts ranging from less than 1 to 18 Kbp were obtained.

Cloning of ILTV DNA. EcoR1 and Bg1II libraries of ILTV DNA were obtained by cloning digests of viral DNA in pUC13 as described above.

Random sequencing of viral DNA. Sonicated fragments of viral DNA were cloned into SmaI-cut, dephosphorylated M13.mp10 (Amersham International PLC) and plaques containing MDV inserts were identified by hybridization to MDV DNA. The sequence was determined by the dideoxy method [Sanger, F. et al (1977) Proc. Natl. Acad. Sci. U.S.A. 74, 5463-5467] using ³⁵S dATP).

The same procedure was used to sequence cloned fragments of MDV, HVT and ILTV DNA except that plaques were identified by hybridization to labelled insert so as to avoid colonies containing pUC13 fragments.

### EXAMPLE 1: gB gene of MDV

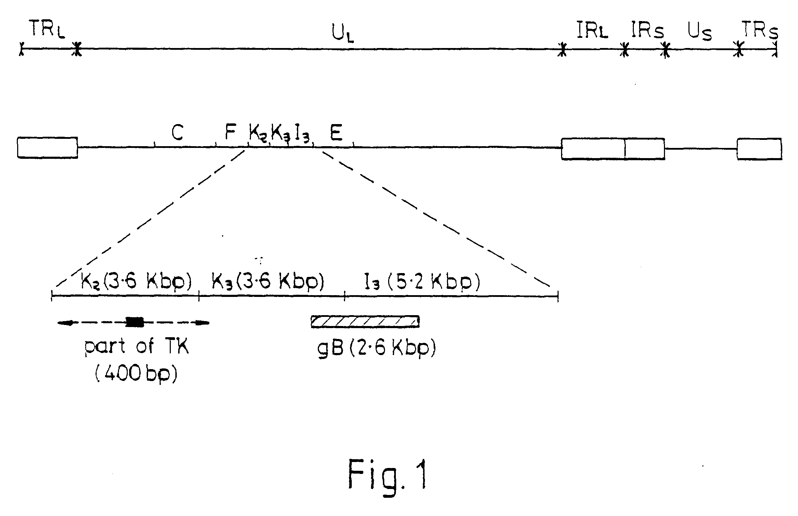

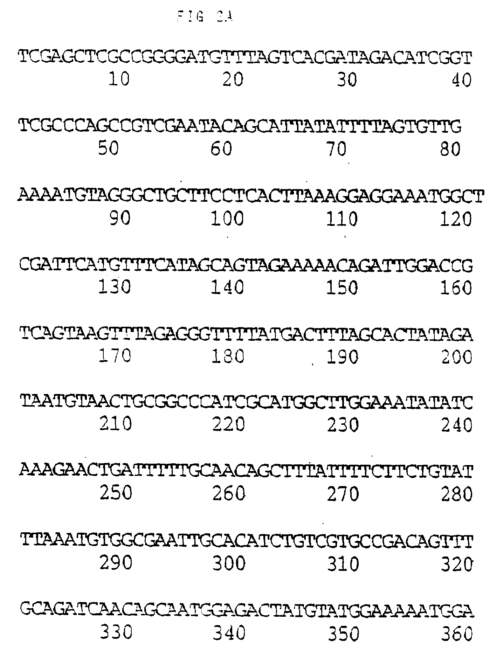

An M13 clone of HVT homologous to the gB gene of VZV and HSV hybridized to BamH1 fragment I3 of MDV (see Figure 1). Sequencing of this fragment obtained from a BamH1 library of the RB1B strain of MDV showed that two thirds of the gene, starting with the NH₂ terminus, was contained within I3. The remainder of the gene was identified in the adjacent restriction fragment K3. Figure 1 shows the map position of the gene which is 2.6Kbp long. Its mRNA has been estimated to be approximately 2.8 Kb. The translated protein is 865 amino acids long (Figure 2). This includes approximately 20 amino acids which may be part of a signal sequence domain. The primary translated sequence of MDV gB has a few features in common with gB of other herpes viruses such as the alignment of cysteine residues and the presence of hydrophobic sequences which are presumably capable of spanning a lipid bilayer [Pellet, P.E. et al (1985), J. Virol. 53, 243-253]. However, MDV gB has only 48% amino acid similarity with gB of HSV and has many unique features such as the insertion of 23 amino acids (residues 1851-1920, Figure 2) and the presence of extra sites with glycosylation potential. Comparison of the sequence of MDV gB with limited sequence data (702 bases) available for HVT gB (Figure 2) has shown 76.9% nucleic acid similarity and 87.1% amino acid similarity between these two glycoproteins. Amino acid substitutions in HVT gB compared to MDV gB were particularly marked in a region (residues 1323 - 1433) equivalent to a domain of HSV gB associated with virus neutralization [Pellet P.E. et al (1985) as above]. Amino acid substitutions between MDV and HVT gB were also noted in other regions of unknown function.

### EXAMPLE 2: TK gene of HVT and TK gene of MDV

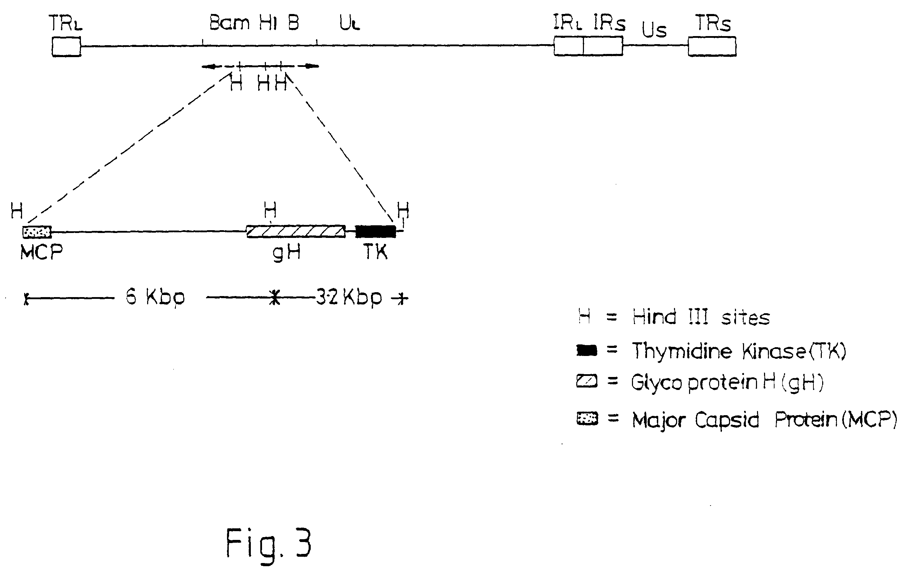

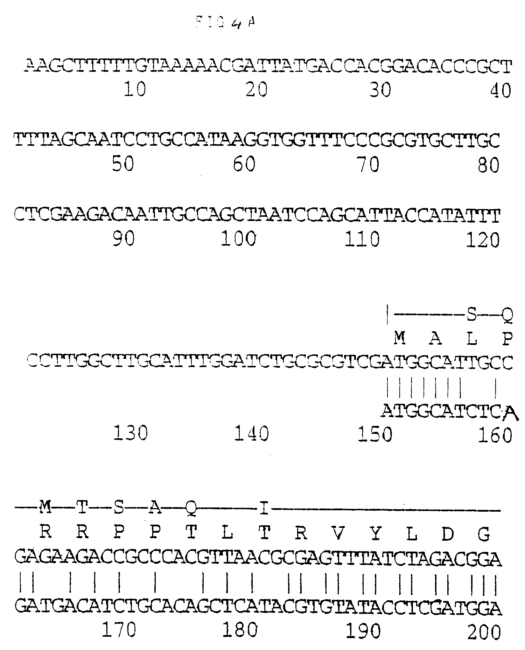

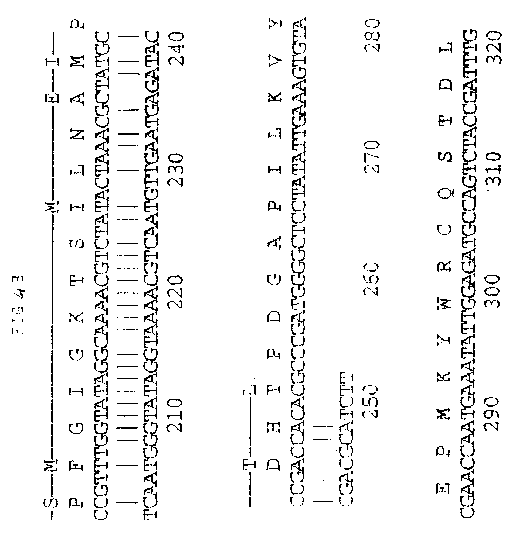

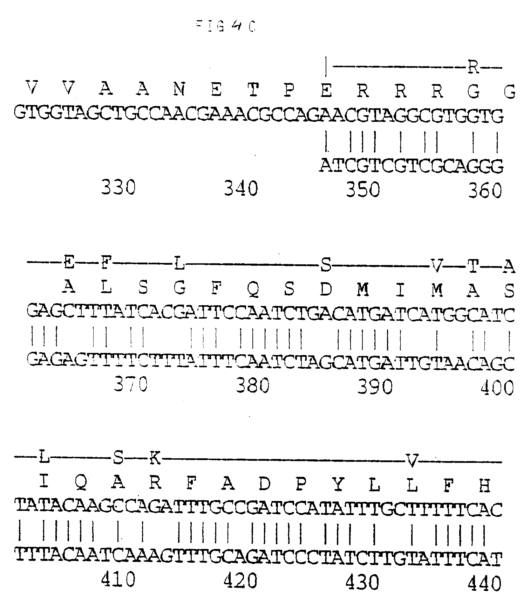

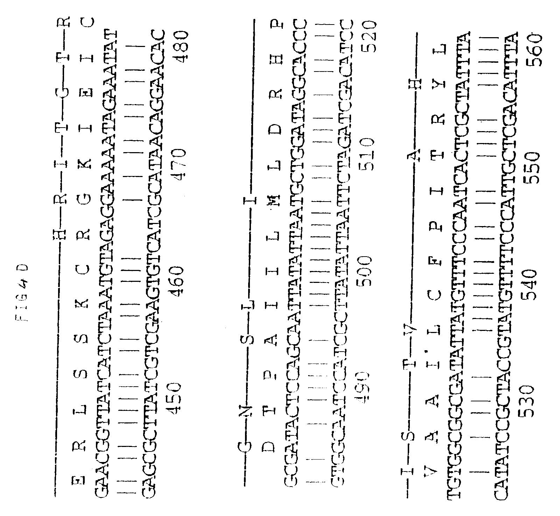

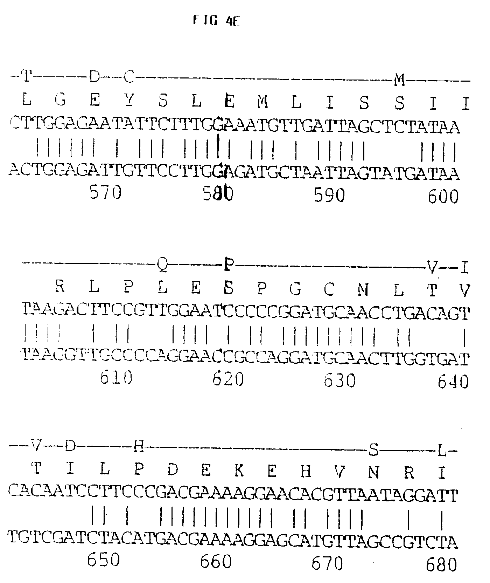

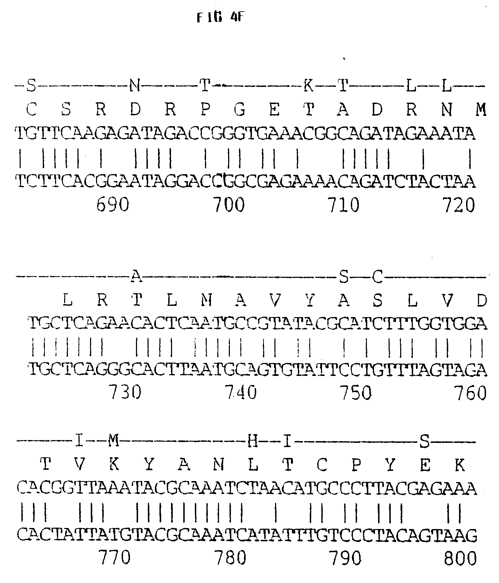

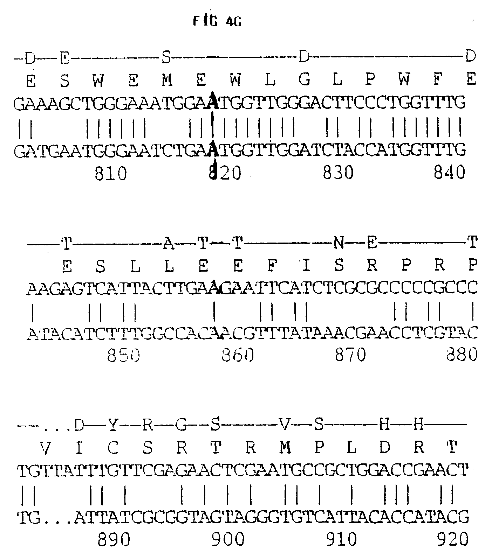

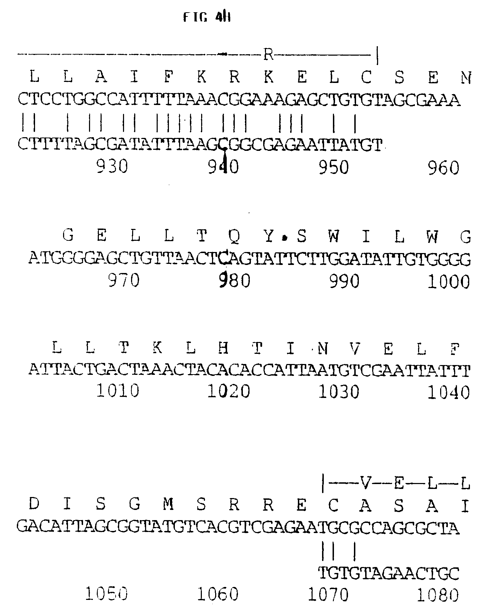

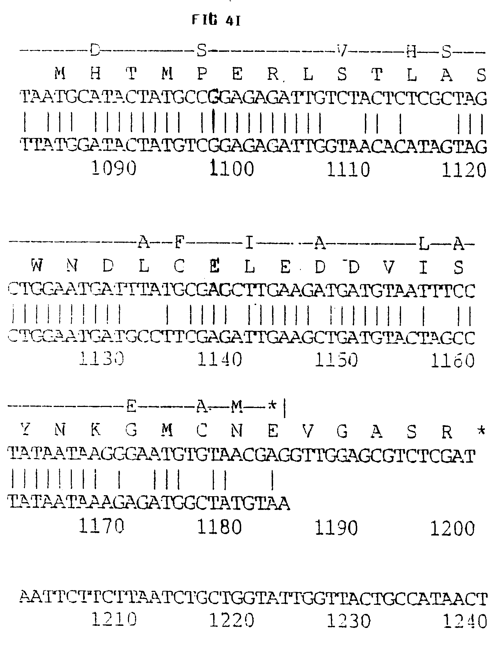

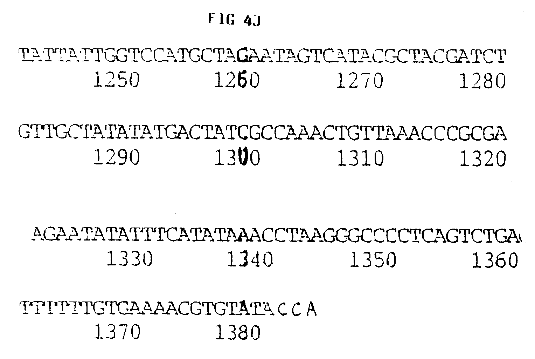

The whole coding region of the TK gene of HVT (1053 bp) was contained within the 3.2 Kbp HindIII fragment described above (Figure 3). The sequence of the entire gene and flanking regions is shown in Figure 4. Similarly the whole of the MDV TK gene is contained within the 3.6 Kbp BamH1 K2 fragment of MDV (Figure 1). The sequence of MDV TK gene determined so far is shown in Figure 4. Comparison of the MDV and HVT TK sequences indicates that the two genes have approximately 60% amino acid identity (estimated from 276 amino acid overlap). By contrast, the % amino acid identities between the TK gene of HVT and the TK genes of HSV 1, VZV and EBV are only 30, 27 and 24 respectively (estimated from amino acid overlaps of 320. 332 and 193 respectively). The predicted amino acid sequences of HVT and MDV TK show characteristic ATP and/or CTP binding site mofifs described for a number of virus and eukaryotic proteins that are associated with phosphorylation (Gentry, G.A. (1985) Proc. Natl. Acad. Sci. U.S.A. 82, 6815-6819). These conserved sequences are examples of useful sites for insertion and expression of foreign genes and for producing TK⁻ deletion mutants.

### EXAMPLE 3: A antigen gene of MDV (gP57-65) (gC homologue)

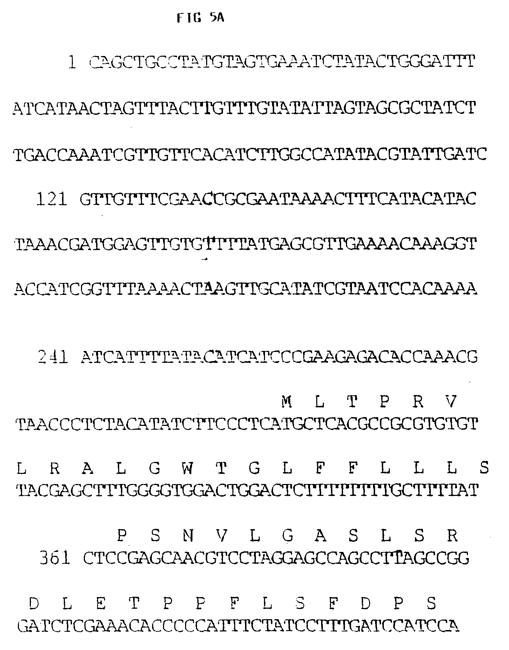

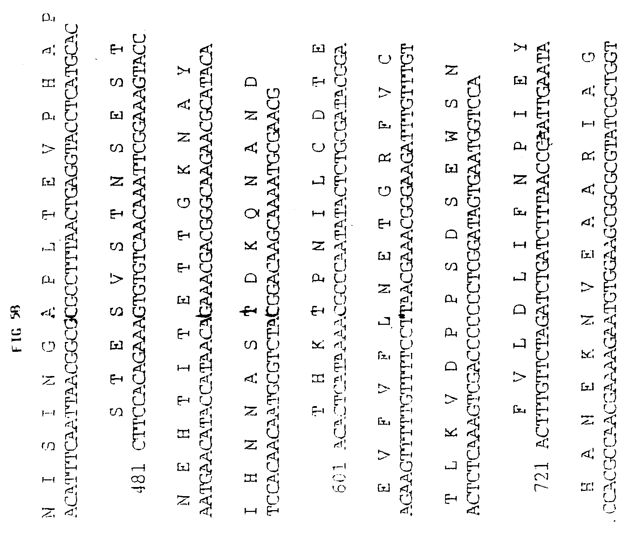

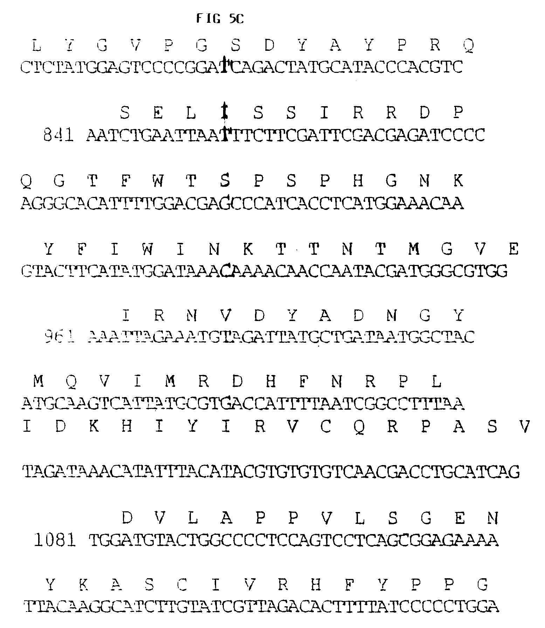

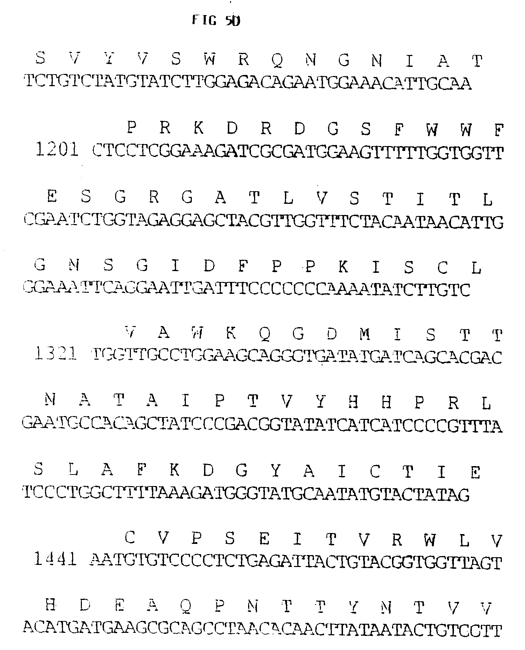

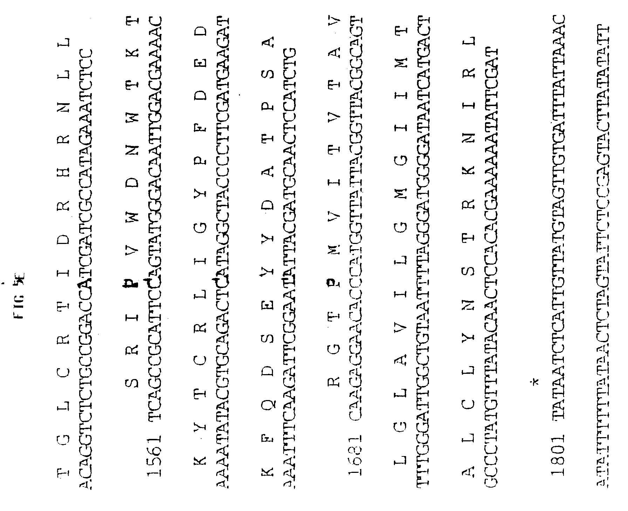

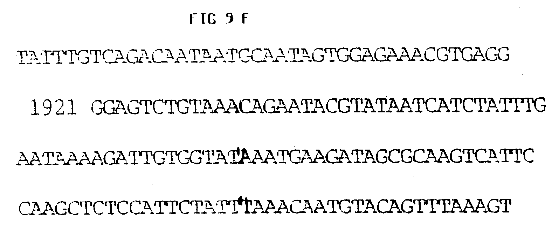

The A antigen gene is of interest in vaccine development because we have identified it as the homologue of HSV gC, a potential non-essential region. The A antigen gene was mapped within the BamH1 B fragment of MDV (Isfort et al 1987), and the nuoleotide sequence determined for the GA strain of MDV (Coussens and Velicer, Abstract OP18.51, VII International Congress of Virology, 9-14 August, (1987) Edmonton, Canada; J. Virol. 62, 2373-2379). During the random sequencing studies described earlier (Buckmaster et al 1988), we identified an M13 clone (No. 130) which came from the A antigen gene. This clone was then used to identify a 2.3 Kbp EcoR1/PvuII fragment from the RB1B strain of MDV containing the A antigen. This fragment was cloned into a SmaI/EcoR1 cleaved pUC13 vector by standard protocols. One plasmid (pMB419) was sequenced by the M13 dideoxynucleotide method. The sequence of the MDV RB1B A antigen and the predicted amino acid sequence of the protein are presented in Figure 5. The A antigen regions of MDV and HTV are non-essential genes and they can therefore be used as sites in MDV and HVT into which other genes can be inserted into the virus by homologous recombination. Several lines of evidence support this as outlined below.
1) During our study we isolated and sequenced another RB1B A antigen clone. This had one extra T residue in the string of T's 45 bases 3' to the A antigen ATG codon. This extra T would cause a frameshift which would make it impossible for the gene to encode functional A antigen. As it is probable that this gene was cloned from a replicating MDV, the results suggest that the A antigen is non-essential to the virus.
2) On conducting a similarity search it became clear that the MDV A antigen gene is the homologue of HSV gC and PRV gpIII glycoproteins. Both of these homologous genes are known to be non-essential [for the HSV homologue, see Rosenthal et al (1987) J. Virol. 61, 2438 - 2447].
3) Strains of MDV lacking A antigen as judged by agar gel diffusion tests [Churchill, A.E. et al (1969) J. gen. Virol. 4, 557-564] or producing low levels using the more sensitive 2D radio-immunoprecipitation (van Zaane, D. et al (1982) Virology 121, 133-146] have been reported.

Furthermore, in view of the fact that the A antigen is a major secreted glycoprotein, it may be a particularly suitable location for the presentation of foreign epitopes within the A antigen as soluble, secreted proteins. This may be achieved by cloning oligonucleotides encoding these epitopes in frame within the A antigen gene.

### STRATEGIES FOR INTRODUCING GENES INTO HVT AND ILTV VECTORS

Two possibilities can be envisaged. 1) Insertion into non-essential genes of the vector. 2) Substitution of foreign gene for corresponding gene of the vector. This would be possible only in regions which already have substantial homology as may be the case between some genes of MDV and HVT.

### EXAMPLE 4: Insertion into non-essential genes of HVT, ILTV or MDV

### (a) Insertion at the TK locus of the vector.

1) HVT, ILTV or MDV may be used as vectors for insertion and expression of avian herpesvirus genes. One may use the promoter associated with the inserted gene or use heterologous promoters, including those of a different class of genes (for example the immediate early promoter to optimise expression of gB).
2) ILTV may be used as a general vector for the insertion and expression of genes unrelated to avian herpes viruses and likely to require manipulation of promoters for optimal expression.

The procedure to be used for gene insertion is substantially as described previously for the insertion of hepatitis antigen in HSV [Shih et al, 1984 as above].

MDV and HVT DNA obtained as described above is infectious provided that precautions are taken not to shear the DNA during extraction. Calcium phosphate precipitates of viral DNA prepared as described by Stow and Wilkie [(1976) J. gen. Virol. 33, 477] were added to sub-confluent monolayers of CEF. After absorption for 1h at 37°C, culture medium was added and cultures were incubated for 1 or 2 days until confluent. Monolayers were then trypsinised, replated (1:1 or 1:2) in 199 medium (Wellcome) containing 2 to 4% calf serum and incubated at 37°C until plaques developed, usually after 4 to 5 days. Approximately 200 plaques may be obtained per µg of HVT DNA and approximately 50 per µg of MDV DNA.

Restriction enzyme sites than could be used for the insertion of foreign antigens into the TK of HVT strain Fc-126 include: BanII, Bsp1286, DraIII, EcoRI, HincII, HpaI, NheI and NspbII.

Some of these enzymes also have sites in the plasmid vector into which the virus DNA fragments are cloned. Thus in order to linearize the clone DNA without also cutting within the vector, partial digests may be carried out.

None of the above enzymes should cause any disruption to flanking gene, HSV-1 homologues of which are known to play an important role in virus multiplication.

For homologous recombination and isolation of recombinant virus, genes of interest are inserted within non-essential genes such as TK or gC and co-transfected with wild-type viral DNA at molar ratios ranging from 10:1 to 2:1 as described above. Alternatively, intact wild-type virus may be used for co-infection.

Virus recombination may be detected by 'plaque lifts' which involve transfer of infected cells and released virus which have adhered to the agar overlay to nitrocellulose and hybridization of the denatured DNA released from the cells and virus to suitable probes as described by Villareal, L. et al (1977) Science 196, 183-185. Virus which hybridizes to the probe may be recovered from the monolayer.

A similar procedure may be used to isolate recombinant virus which expressed epitopes of interest. In this instance the nitrocellulose "plaque lifts" are treated with antibody and the presence of bound antibody revealed using a suitable detection system such as labelled protein A or phosphatase conjugated anti-globulin antibody.

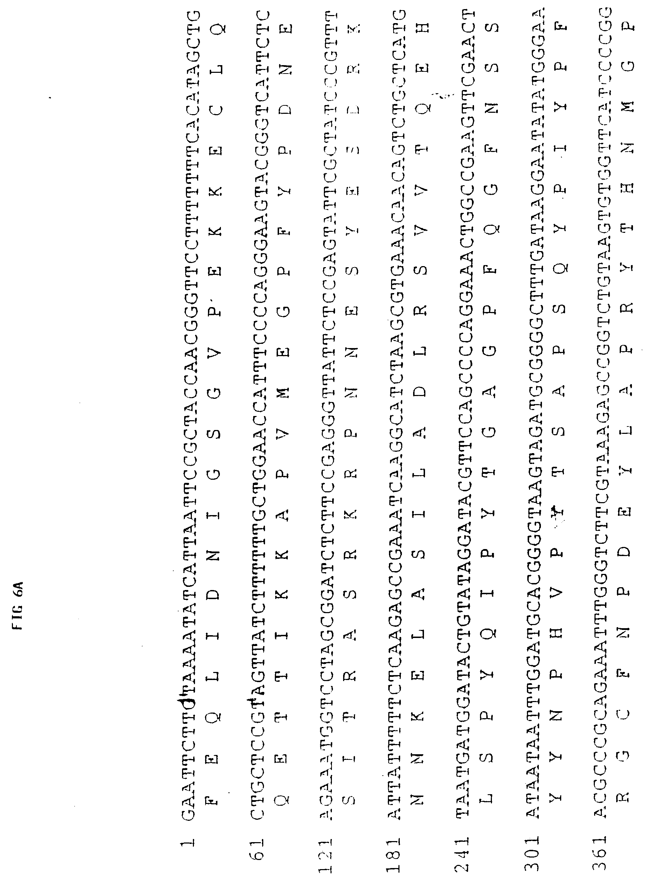

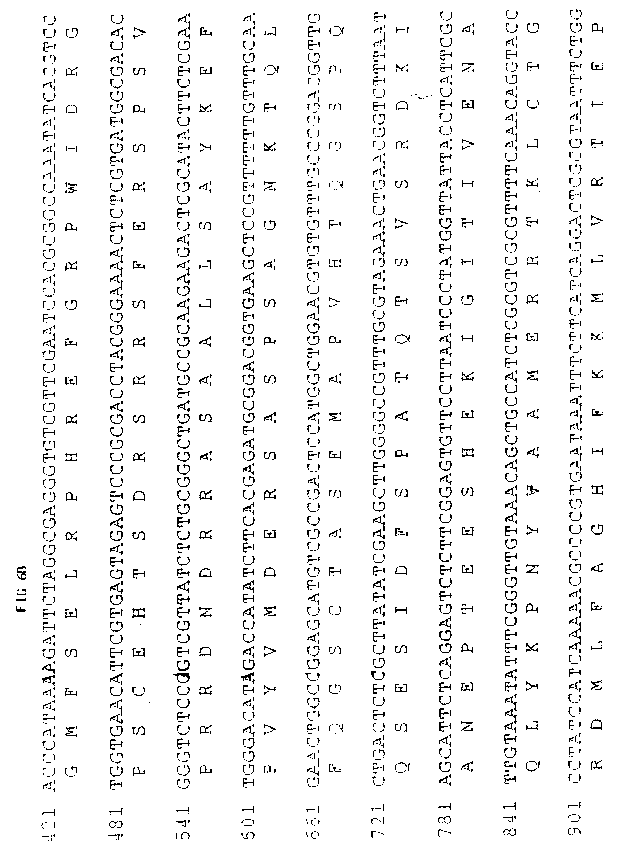

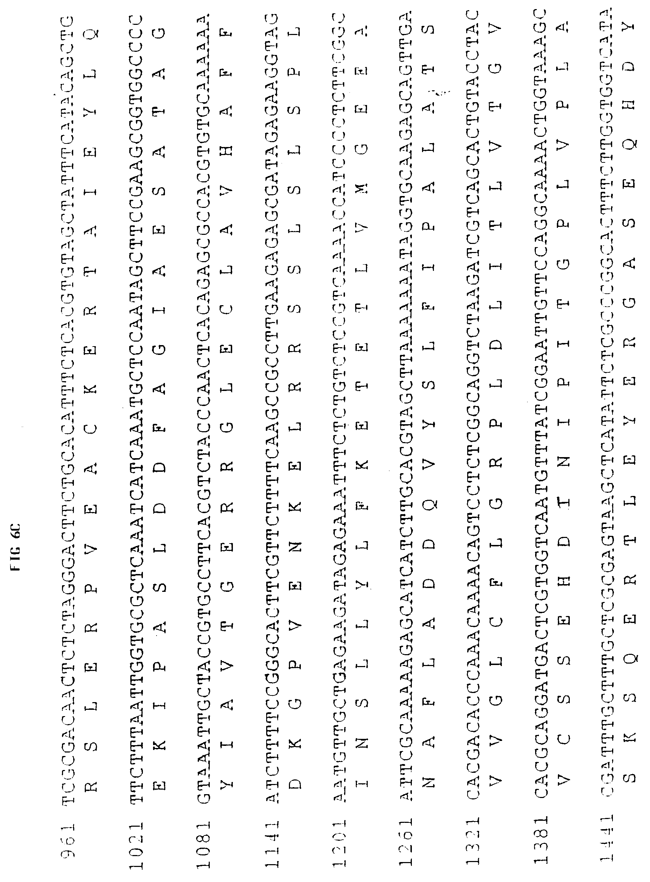

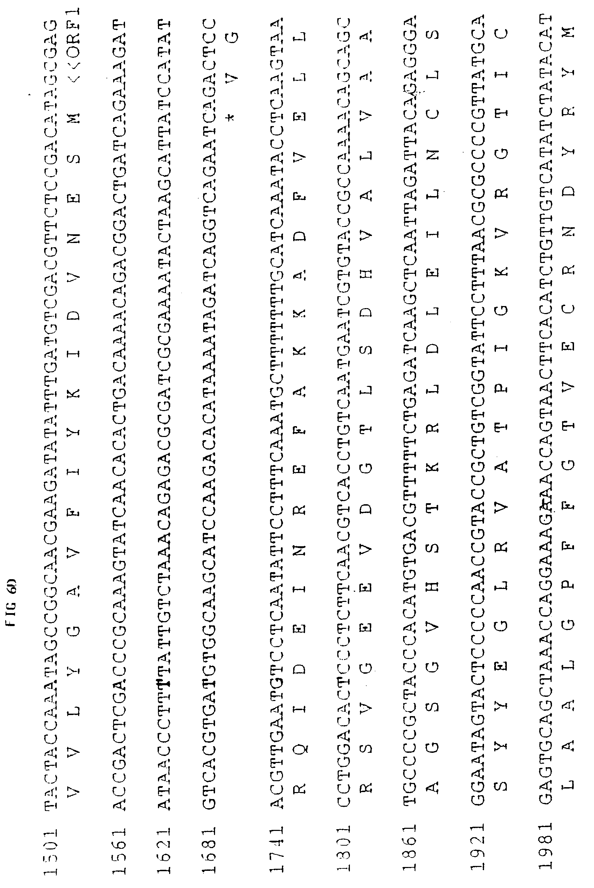

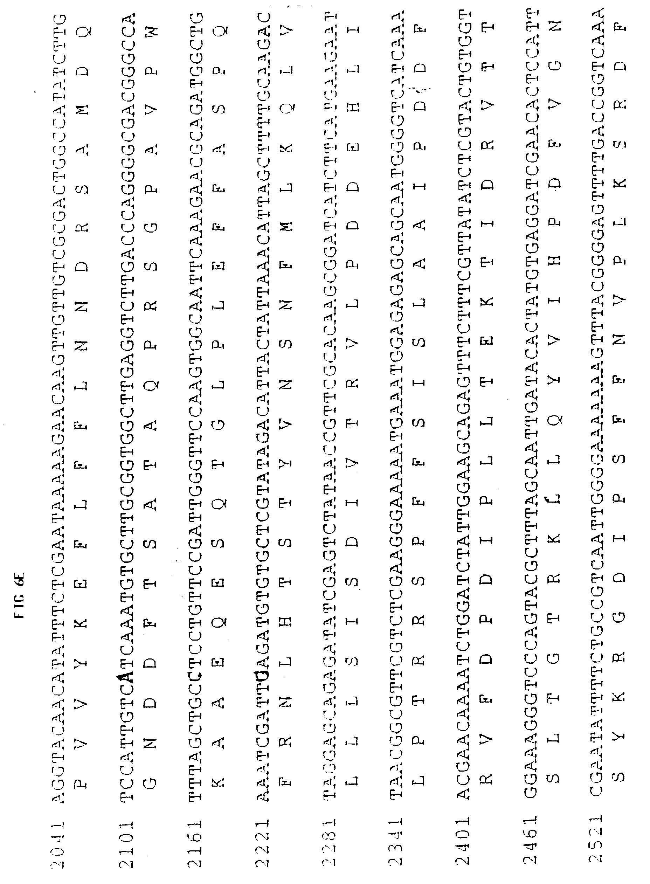

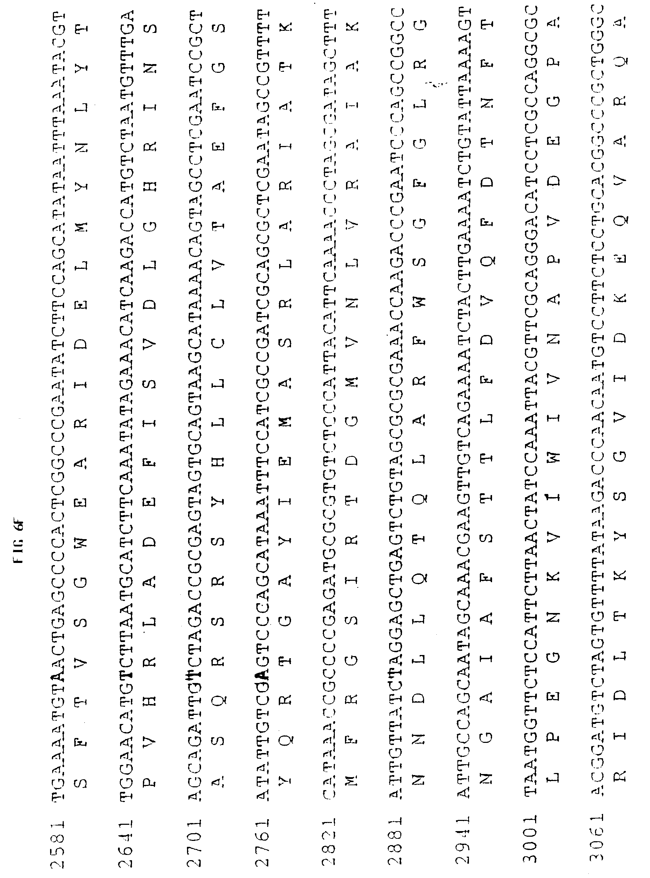

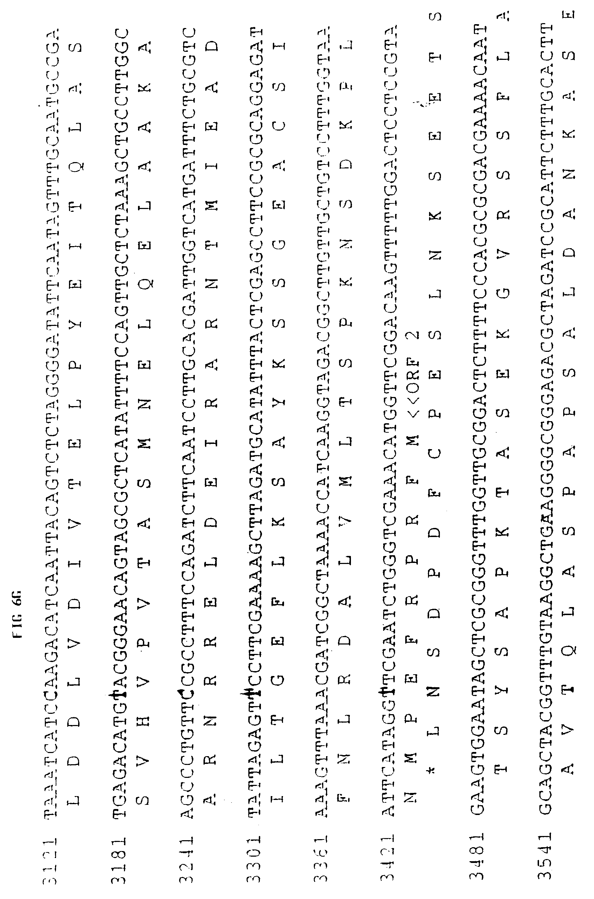

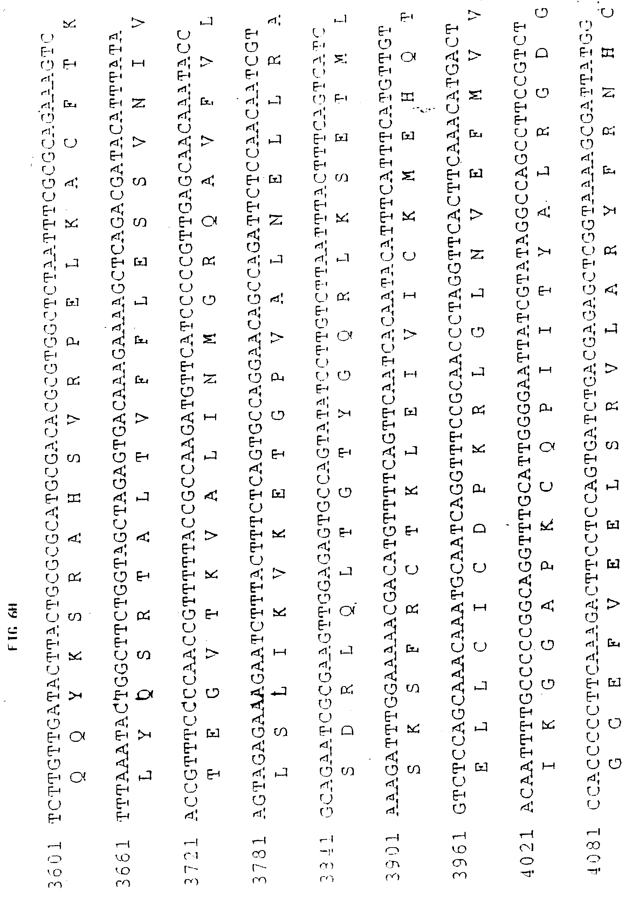

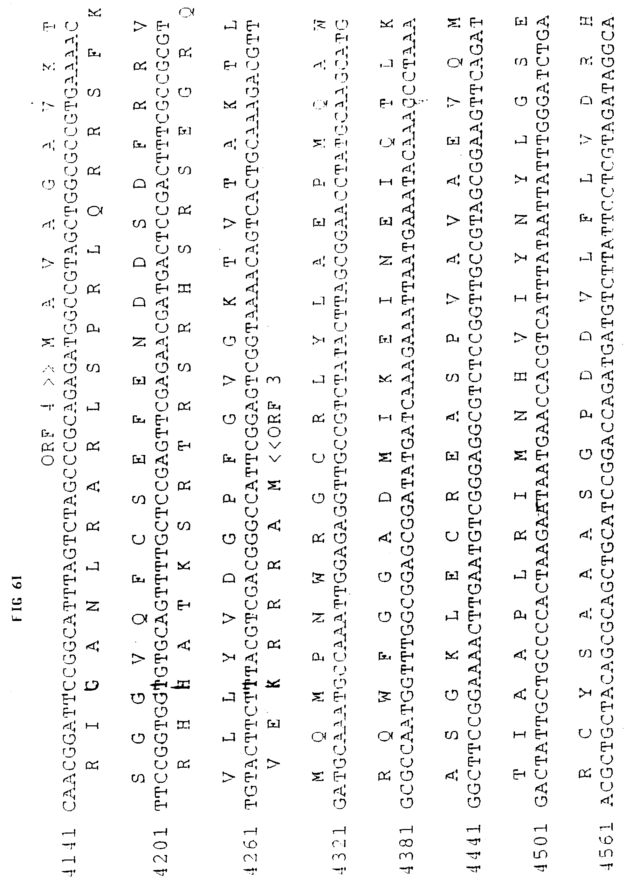

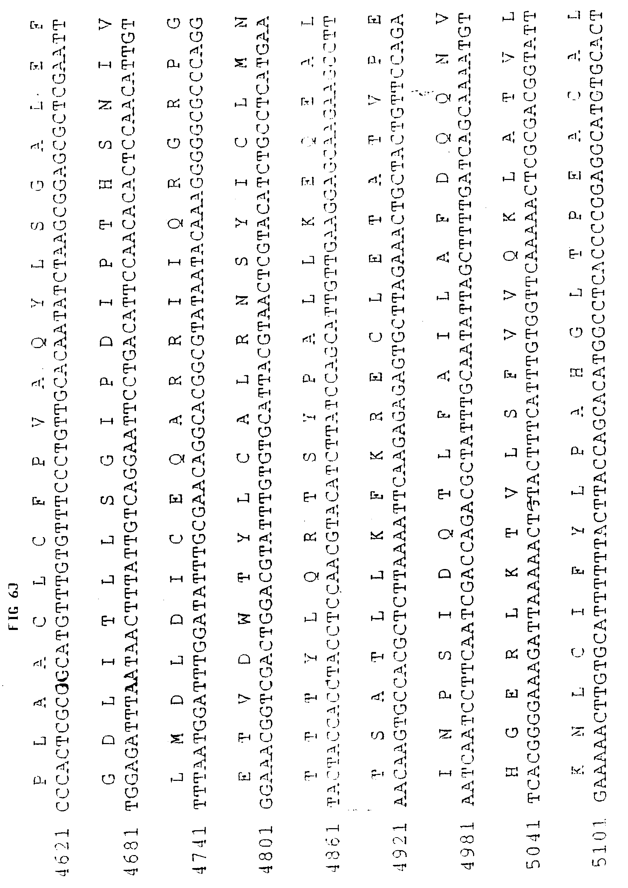

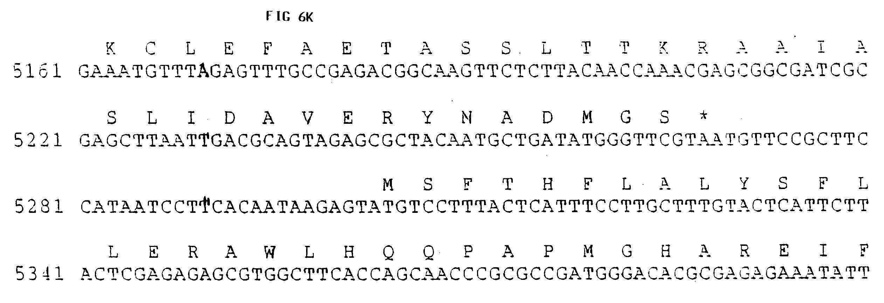

The gene of interest with appropriate premoters is first inserted within the cloned TK gene (Figure 6). The recombinant DNA is then co-transfected with infectious DNA of the vector in chick embryo fibroblasts or chicken kidney cells and TK⁻ virus may be selected by growth in medium containing acyclovir [Ross, N. (1985) as above] or FMAU [Schat, K.A. et al (1984) Antiviral Research 4, 159-270]. Alternatively, or in addition, plaques are screened for the presence of the gene of interest using 'plaque lifts' on nitrocellulose and hybridization to any relevant labelled probe. Plaques are also screened for expression of the epitopes of interest using monoclonal antibodies or antipeptide antibodies.

The main advantage of this strategy is that the selection procedure increases the chances of obtaining virus recombinants containing the gene of interest. It also offers the opportunity of using different promoters for optimum expression. Thus the use of an immediate early promoter may allow expression in latently infected cells.

### (b) Insertion at the gC locus of the vector.

Since the A antigen (HVT and MDV homologues of HSV gC) is not essential for virus growth in vivo and in vitro (see section on gC above) it is a potentially useful site for the insertion and expression of foreign genes. Moreover, since it is one of the most abundant antigens and is excreted, it may be particularly useful for enhancing the immunogenic properties of foreign proteins. The isolation of virus recombinants at this locus may be achieved by first inserting at least part of the gene of interest in frame within the gC gene and then co-transfecting with infectious viral DNA. Screening of virus plaques with sequence specific probes or with specific antibody allows the isolation of recombinants.

### EXAMPLE 5: Substitution of ILTV genes for their homologues in HVT

Substitution may be achieved by co-transfection of cloned ILTV sequences and infeetious HVT DNA as described in Example 4. Substitution of genes derived from ILTV for their counterparts in HVT may be effected.

Recombinants expressing ILTV sequences and epitopes may be detected using ILTV-specific monoclonal antibodies or anti-peptide antibodies raised against unique ILTV sequences as described above.

The advantage of this procedure is that it is relatively simple and does not require manipulation of promoters. However, it may be limited to genes which share substantial homology.

### EXAMPLE 6: Strategies for obtaining TK⁻ mutants of ILTV

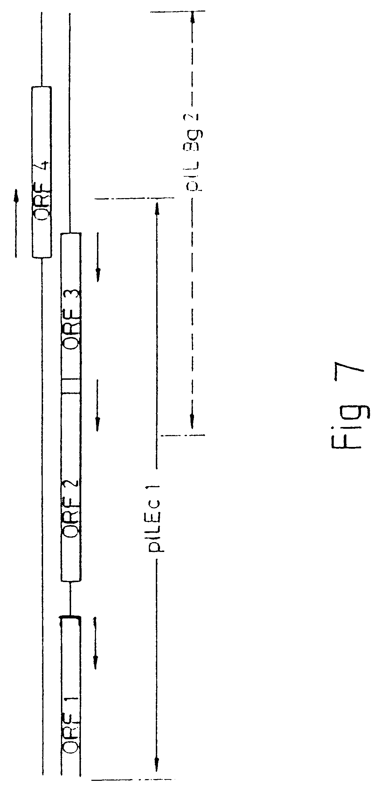

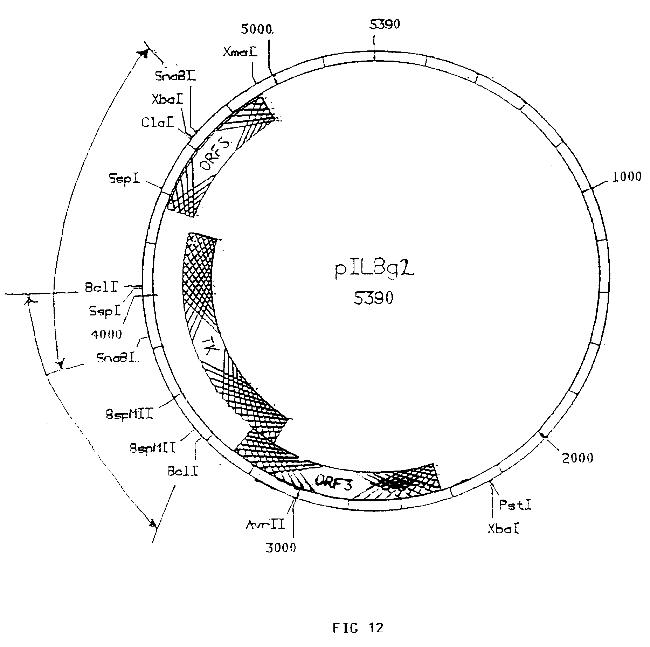

Deletion mutants. Deletions may be introduced within any suitable part of the gene, for example the domains of the gene that are required for its function as a phosphorylating enzyme such as ATP and CTP binding sites. This may be achieved by restriction enzyme digestion, for example with SnaB1 or BclI, and religation of appropriate fragments followed by co-transfection with infectious viral DNA or transfection into virally-infected cells. Reference may be made to Figures 6 and 7, and to the map of plasmid pILBg2 (Figure 12), in choosing restriction enzymes and so on. TK⁻ virus may be selected in the presence of acyclovir [Ross, N. (1985) as above] or FMAU [Schat, K.A. et al (1984) as above]. Plaque-purified clones may then be tested for the absence of the deleted portion of the TK gene by hybridization.

The deletion mutants of ILTV may be used themselves as attenuated viruses for vaccine preparation, or may have sequences for heterologous antigens inserted.

Insertional mutants. A functional β-galactosidase gene under the control of a herpesvirus promoter or any other suitable sequence or a single base is first introduced in a domain of the TK gene which is essential for TK activity. The recombinant DNA is then co-transfected with infectious viral DNA or transfected into virally-infected cells to allow homologous recombination to occur. Selection in the presence of acylovir or FMAU will yield TK⁻ insertional mutants. If a β-galactosidase gene is introduced, mutants can be detected by the production of blue plaques in the presence of X-gal.

The TK gene and surrounding sequences may be subcloned into another suitable vector if necessary.

### EXAMPLE 7: Insertion of MDV RB1B gB gene into HVT

(Not within the scope of the invention, but illustrates an analogous technique).

The HVT TK gene is cloned in the plasmid vector pUC13 to generate a plasmid, which may be termed pTK1B. This plasmid is linearised with, for example, the restriction endonuclease Rsr II which cleaves the plasmid only within the TK gene (nucleotide position 197 in Figure 4, enzyme recognition sequence CGGACCG). The "sticky" ends thus generated are end repaired by standard techniques (see "Molecular Cloning: a Laboratory Manual", ed. Maniatis T., Fritsch E.F., and Sambrook J. Cold Spring Harbor Laboratory 1982).

The RB1B gB was originally cloned on two plasmids which were termed RB1B-BamH1-I₃ and RB1B-BamH1-K₃. (Note I₃ had lost one BamH1 site during cloning.) To generate a complete gB copy on one plasmid, both plasmids were cleaved with BamH1 and the fragments ligated. However, the complete gB gene was later obtained independently on an EcoRI/SalI fragment. Ross et al, J. gen. Virol (1989) 70, 1789-1804 provides further information regarding the manipulation of viral genes. Recombinants containing the desired configuration can be identified by restriction enzyme analysis of plasmid DNA's.

The recombinant plasmid is then cleaved with EcoR1, the ends are repaired and the plasmid is cloned into PTK1B prepared as above. The recombinant plasmid is then introduced into cells containing HVT virus (viral DNA) and homologous recombination will introduce the gB gene into the TK gene. HVT viral recombinants can be selected with acyclovir or FMAU or alternatively detected with labelled gB probes.

In analogous ways, the sequence information given above and in the Figures can be used to design cloning strategies for the insertion of the DNA fragment according to the invention into the non-essential genes of the ILTV described here or to generate combinations of antigen genes into ILTV.

## Claims

1. A DNA fragment encoding the HVT gB gene which comprises the HVT nucleotide sequence portions of Figure 2 indicated between nucleotide positions 890-1081, 1172-1445 and 2190-2426 or minor variations thereof not affecting the essential nature of said fragment.

2. A DNA fragment according to Claim 1, characterized in that it comprises the coding portion of the gB gene sequence and at least part of the 5' and/or 3' non-coding portions thereof.

3. A plasmid vector comprising a DNA fragment according to Claim 1 or 2 and a DNA portion allowing replication in a suitable host.

4. A plasmid vector according to Claim 3 which is suitable for transfection of an MDV-, HVT- or ILTV-susceptible cell.

5. A viral vector comprising a DNA fragment according to claim 1 or 2.

6. A viral vector according to claim 5, characterized in that the virus used as vector is a poxvirus, preferably a fowlpoxvirus.

7. A mutant MD or ILT virus comprising a DNA fragment according to Claim 1 inserted in the gC, ribonucleotide reductase (small subunit) or ribonucleotide reductase (large subunit) gene of MDV or in the TK, ORF2, ORF3 or ribonucleotide reductase (large subunit) gene of ILTV, or a mutant HVT virus comprising an extra copy of the DNA fragment of Claim 1 inserted in the gC, ribonucleotide reductase (large subunit) or TK gene of HVT.

8. A vaccine comprising ILTV-susceptible cells and a mutant ILT virus according to Claim 7 such that the virus is attenuated, at least partially as a result of such mutation.

## Patentansprüche

1. DNA-Fragment, codierend das HVT-gB-Gen, welches die HVT-Nukleotid-Sequenz-Bereiche von Figur 2 umfasst, die zwischen den Nukleotid-Positionen 890-1081, 1172-1445 und 2190-2426 angegeben sind, oder geringfügige Varianten davon, die die essenrielle Natur des Fragmentes nicht beeinträchtigen.

2. DNA-Fragment nach Anspruch 1, dadurch gekennzeichnet, dass es den codierenden Bereich der gB-Gensequenz und zumindest einen Teil der 5'- und/oder 3'-nicht-codierenden Bereiche davon umfasst.

3. Plasmid-Vektor, umfassend ein DNA-Fragment nach Anspruch 1 oder 2 und einen DNA-Abschnitt, der die Replikation in einem geeigneten Wirt ermöglicht.

4. Piasmid-Vektor nach Anspruch 3, der sich zur Transfektion einer MDV-, HVT- oder ILTV-empfindlichen Zelle eignet.

5. Vitaler Vektor, umfassend ein DNA-Fragment nach Anspruch 1 oder 2.

6. Viraler Vektor nach Anspruch 5, dadurch gekennzeichnet, dass das als Vektor verwendete Virus ein Pockenvirus, vorzugsweise ein Vogelpockenvirus, ist.

7. Mutiertes MD- oder ILT-Virus, umfassend ein DNA-Fragment nach Anspruch 1, das inseriert ist in das Gen für gC-, Ribonukleotidreduktase (kleine Untereinheit) oder Ribonukleoridreduktase (große Untereinheit) von MDV oder in das Gen für TK, ORF2, ORF3 oder Ribonukleotidreduktase (große Untereinheit) von ILTV, oder ein mutiertes HVT-Virus, umfassend eine zusätzliche Kopie des DNA-Fragmentes nach Anspruch 1, das inseriert ist in das Gen für gC-, Ribonukleotidreduktase (große Untereinheit) oder das TK-Gen von HVT.

8. Impfstoff, umfassend ILTV-empfindliche Zellen und ein mutiertes ILT-Virus nach Anspruch 7, so dass das Virus - zumindest teilweise als Folge dieser Mutation - abgeschwächt ist.

## Revendications

1. Fragment d'ADN codant pour le gène gB de HVT qui comprend les portions de séquence nucléotidique HVT de la figure 2, indiquées entre les positions des nucléotides 890-1081, 1172-1445 et 2190-2426 ou leurs variations mineures, n'affectant pas la nature essentielle dudit fragment.

2. Fragment d'ADN selon la revendication 1, caractérisé en ce qu'il comprend la portion codante de la séquence du gêne gB et au moins une partie de ses portions non codantes en 5' et/ou 3'.

3. Vecteur plasmidique comprenant un fragment d'ADN selon la revendication 1 ou 2, et une portion d'ADN permettant une réplication dans un hôte approprié.

4. Vecteur plasmidique selon la revendication 3, qui convient à la transfection d'une cellule sensible à MDV, HVT ou ILTV.

5. Vecteur viral comprenant un fragment d'ADN selon la revendication 1 ou 2.

6. Vecteur viral selon la revendication 5, caractérisé en ce que le virus utilisé comme vecteur est un poxvirus, de préférence un fowlpoxvirus.

7. Virus MD ou ILT mutant, comprenant un fragment d'ADN selon la revendication 1 inséré dans le gêne gC, de la ribonucléotide réductase (petite sous-unité) ou de la ribonucléotide réductase (grande sous-unité) de MDV ou dans le gène TK, ORF2, ORF3 ou de la ribonucléotide réductase (grande sous-unité) d'ILTV, ou un virus HVT mutant comprenant une copie supplémentaire du fragment d'ADN de la revendication 1 inséré dans le gène gC, de la ribonucléotide réductase (grande sous-unité) ou TK de HVT.

8. Vaccin comprenant des cellules sensibles à ILTV et un virus ILT mutant selon la revendication 7, tel que le virus soit atténué, au moins partiellement, du fait de cette mutation.
